# EUROPEAN PATENT SPECIFICATION

(11) **EP 2 679 249 B1**
(45) Date of publication and mention of the grant of the patent: **01.02.2017**
(21) Application number: 13162321.7
(22) Date of filing: 04.04.2013
(51) Int. Cl.: A61L 9/03

(54) **Aroma-diffusing heating device using a replaceable aroma capsule and the aroma capsule**
Aromaverteilende Heizungsvorrichtung, die eine austauschbare Aromakapsel verwendet, und Aromakapsel
Dispositif de chauffage diffusant un parfum utilisant une capsule parfumée remplaçable et ladite capsule

(30) Priority: 28.06.2012 CN 201220308493 U
(43) Date of publication of application: 01.01.2014
(73) Proprietor: Dongguan Yih Teh Electric Products Co., Ltd, Dong Guan, Guangdong (CN)
(72) Inventor: WIRZ, Peter, 6000 Luzern (CH)
(74) Representative: Viering, Jentschura & Partner mbB Patent- und Rechtsanwälte

(56) References cited:
- EP-A1- 1 473 046
- EP-A1- 1 627 647
- EP-A1- 2 067 491
- EP-A2- 0 321 729
- AU-A4- 2012 101 327
- US-A- 6 085 026

## Description

### 1. Field of the Invention:

The present invention relates to scent releasing devices and more specifically, to an aroma-diffusing heating device that uses a replaceable aroma capsule. The invention relates also to the aroma capsule.

### 2. Description of the Related Art:

In modern society, in order to remove the smell in the air, or to enrich the spice of life, many people started using fragrance products (for example, aromatic wax, essential oils). When in use, a user needs to remove the aromatic wax from the aromatic wax container and put it in an accommodation chamber in a heater, and then electrically conduct the heater to heat the aromatic wax, causing the aromatic wax to give off fragrance.

The aforesaid application method has drawbacks. During application, the user must remove the aromatic wax from the aromatic wax container and then put the aromatic wax in the accommodation chamber in the heater carefully, avoiding direct contact of the hands with the aromatic wax. After the aromatic wax having been put in the accommodation chamber in the heater, the aromatic wax may be forced out of the heater accidentally upon an impact. Further, after the aromatic wax in the accommodation chamber in the heater is used up, the user needs to clean the accommodation chamber. If the accommodation chamber is not cleaned after each use, a new supply of aromatic wax will be mixed with the residual aromatic wax in the accommodation chamber, giving a bad smell. Further, if the accommodation chamber is formed of a fragile material (for example, ceramics), it may be broken easily during cleaning. An electrically heated device for dispensing fragrancing materials is known from US 6 085 026.

Therefore, it is desirable to provide an aroma-diffusing design that eliminates the drawbacks of the aforesaid prior art design.

### SUMMARY OF THE INVENTION

The present invention has been accomplished under the circumstances in view. It is therefore the main object of the present invention to provide an aroma-diffusing heating device as defined in independent claim 1, which is configured to heat an aroma capsule to give off fragrance, wherein the aroma capsule is replaceable with a new one when used up.

To achieve this and other objects of the present invention, an aroma capsule, comprising: a heat-transfer container defining a top opening; an aromatic substance held in said heat-transfer container is provided.

In an embodiment, the aroma capsule further comprises a sealing film bonded to the heat-transfer container to seal the aromatic substance in the heat-transfer container, maintaining the quality of the aromatic substance.

In another embodiment, the aroma capsule further comprises a sealing film; and said heat-transfer container comprise a rim around the top opening; the sealing film bonded to the rim of the heat-transfer container to seal the aromatic substance in the heat-transfer container, maintaining the quality of the aromatic substance.

In an embodiment, the aroma capsule further comprises a breathing film having open spaces and bonded to the heat-transfer container above the aromatic substance.

In an embodiment, the breathing film further comprises a border edge, said breathing film bonded with the border edge thereof to the heat-transfer container above the aromatic substance .

In an embodiment, the breathing film further comprises a border edge; the heat-transfer container further comprise a rim around the top opening; said breathing film bonded with the border edge thereof to the rim of the heat-transfer container above the aromatic substance.

In an embodiment, further compring a sealing film bonded to the heat-transfer container to seal the aromatic substance and the breathing film in the heat-transfer container, maintaining the quality of the aromatic substance.

In an embodiment, the heat-transfer container further comprises a rim around the top opening; the sealing film bonded to the rim of the heat-transfer container.

In an embodiment, further compring a sealing film bonded to the rim of the heat-transfer container to seal the aromatic substance and the breathing film in the heat-transfer container, maintaining the quality of the aromatic substance.

In an embodiment, the breathing film is selected from the material group consisting of nonwoven fabric, fiber cloth, woven fabric, porous plastic membrane and porous silicon rubber, and has the border edge thereof bonded to said container in a top opening of said container.

In an embodiment, the container comprises a rim, and said sealing film is bonded to said rim of said container, and is made of metal (e.g., aluminum), ceramic, or plastic.

According to the invention, the aroma substance is an aromatic wax.

According to the invention, the aroma capsule is applied to an aroma-diffusing heating device having a heating base defining a top accommodation open chamber for the aroma capsule to be accommodated therein, and the heating base comprises a top flange raised from a top wall thereof around said top accommodation open chamber, said top flange having an inside wall thereof smoothly downwardly curved toward said top accommodation open chamber.

### BRIEF DESCRIPTION OF THE DRAWINGS

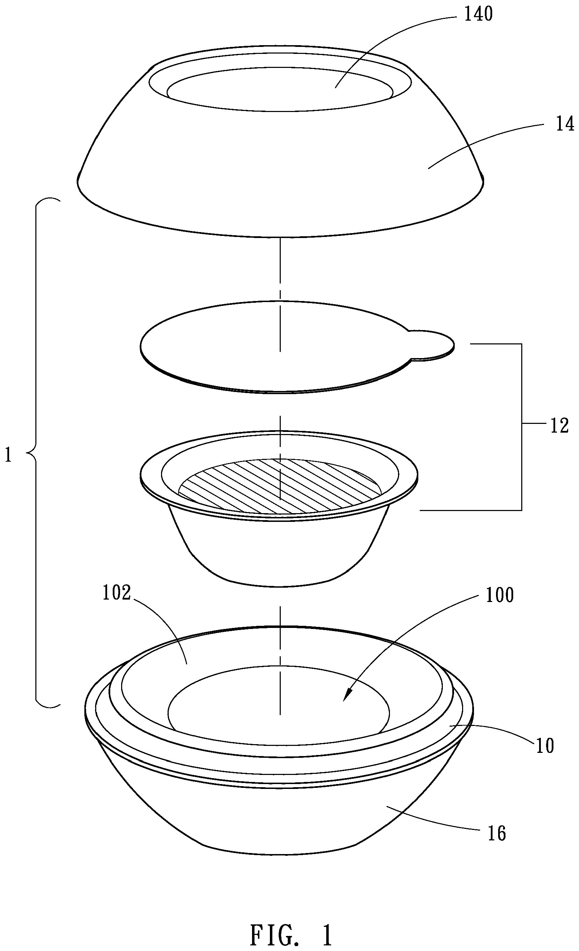

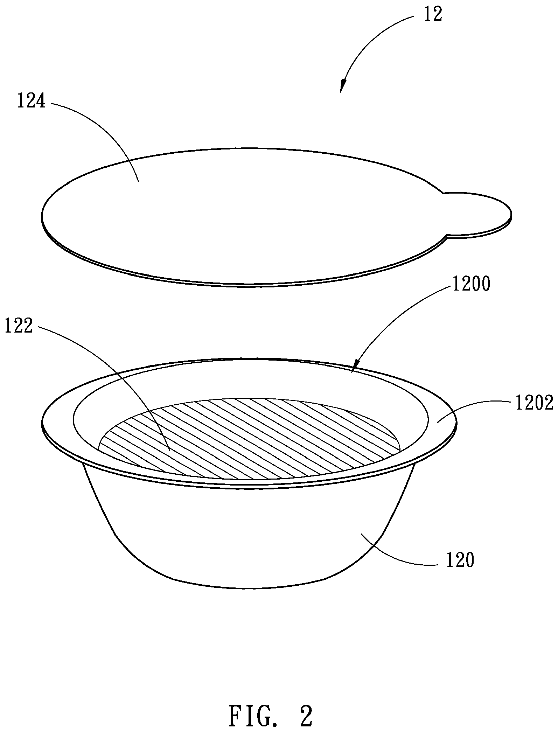

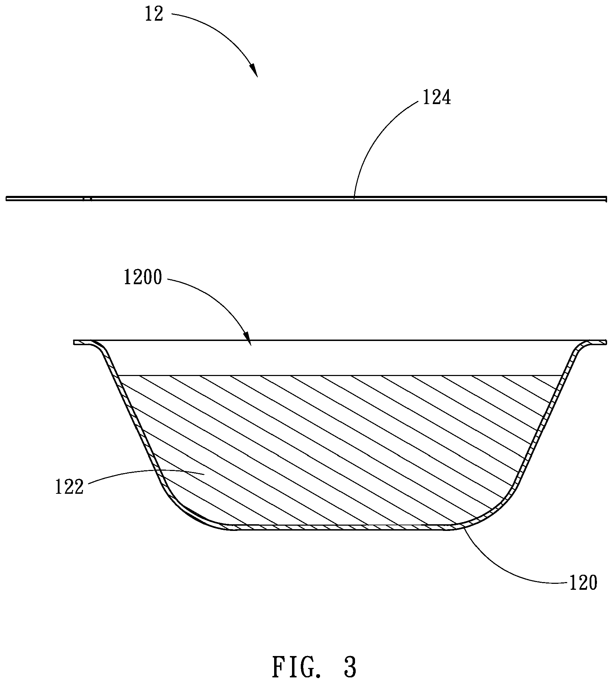

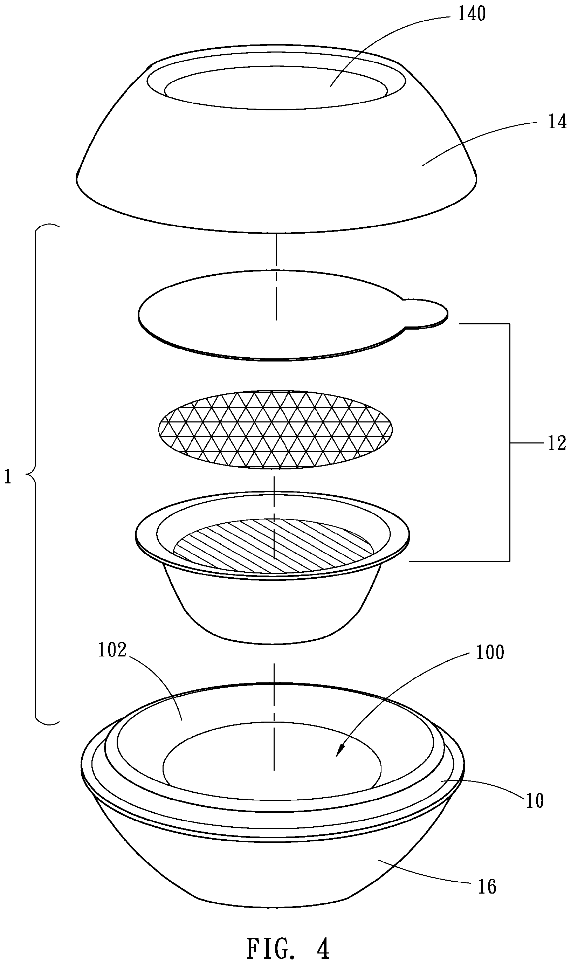

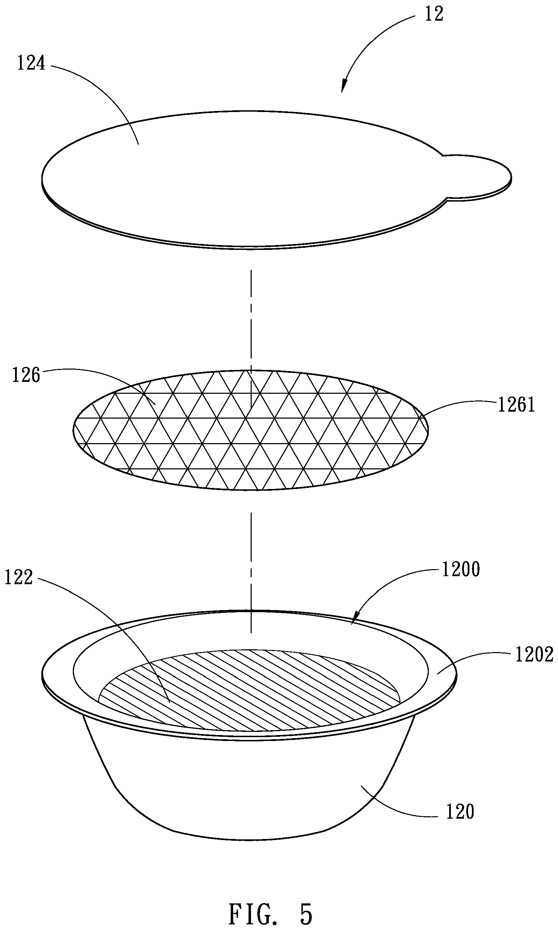

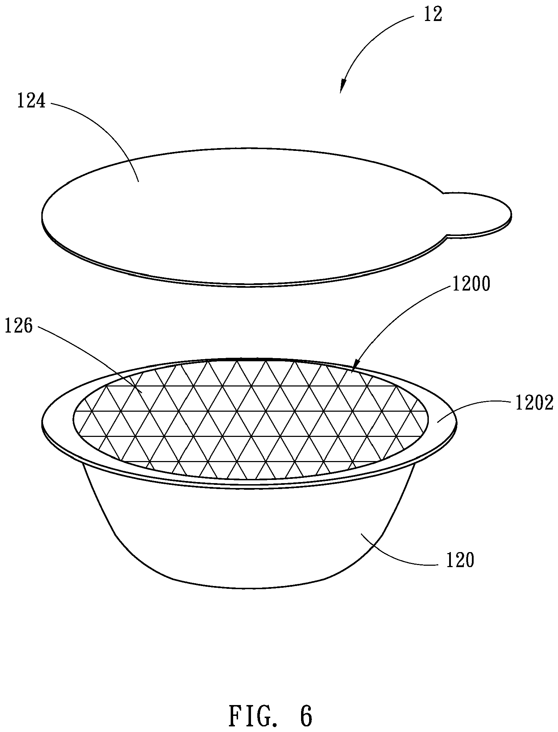

FIG. 1 is an exploded view of an aroma-diffusing heating device in accordance with the present invention. FIG. 2 is an exploded view of the aroma capsule of the aroma-diffusing heating device in accordance with the present invention. FIG. 3 is a sectional side view of FIG. 2. FIG. 4 is an exploded view of an alternate form of the aroma-diffusing heating device in accordance with the present invention. FIG. 5 is an exploded view of the aroma capsule shown in FIG. 4. FIG. 6 corresponds to FIG. 5, illustrating the breathing film sealed to the heat-transfer container.

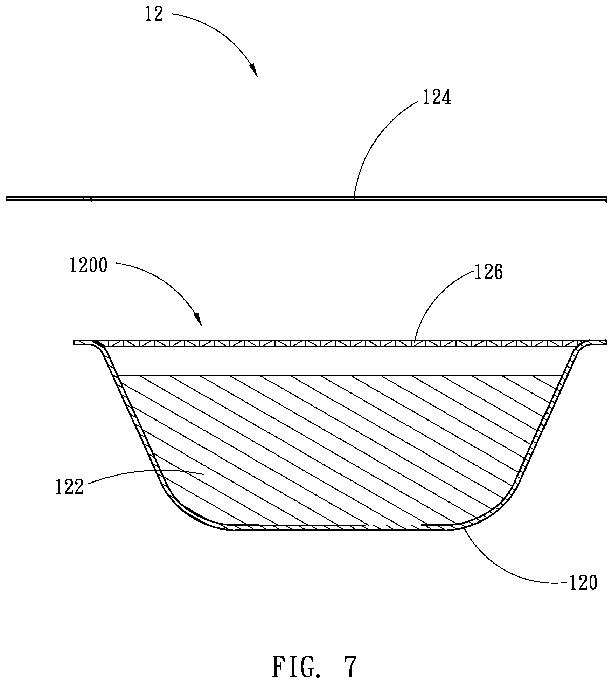

FIG. 7 is a sectional side view of FIG. 6.

### DETAILED DESCRIPTION OF THE PREFERRED EMBODIMENT

Referring to FIGS. 1-3, an aroma-diffusing heating device **1** in accordance with the present invention is shown having an aroma capsule **12** therein. The aroma-diffusing heating device **1** comprises a ceramic bowl **16,** a heating base **10** supported on the ceramic bowl **16** and defining a top accommodation open chamber **100** that accommodates the aroma capsule **12,** and a cap **14** capped on the heating base **10** over the aroma capsule **12.**

The aroma capsule **12** comprises a heat-transfer container **120** kept in contact with the heating base **10** and defining a top opening **1200,** an aromatic substance **122** (for example, aromatic wax in this preferred embodiment, essential oil, or perfume) put in the heat-transfer container **120,** and a sealing film **124** bonded to the heat-transfer container **120** to seal the aromatic substance **122** in the heat-transfer container **120,** maintaining the quality of the aromatic substance **122.**

During application, the user simply needs to remove the sealing film **124** from the rim **1202** of the heat-transfer container **120** and then electrically conducting the heating base **10** to heat the aromatic substance **122,** causing the aromatic substance **122** to give off fragrance into the outside open air.

According to the present invention, the heating base **10** is configured to heat the aromatic substance **122** to about 40∼70°C, or preferably 50∼55°C. Within this temperature range, commercial aromatic substances can give off fragrance without destroying the quality. Further, under this temperature range of 40∼70°C, the heat-transfer container **120** is maintained intact and can effectively transfer heat from the heating base **10** to the aromatic substance **122**(aromatic wax). The heat-transfer container **120** can be selected from the material group of ceramics, metals, and hard plastics. Preferably, the heat-transfer container **120** is made from a light metal, for example, aluminum foil for the advantages of light weight, high toughness and high thermal conductivity, avoiding the disadvantage of high fragileness of pottery bowls and glass containers. Further, the sealing film **124** seals the aromatic substance **122** in the top opening **1200,** maintaining the quality of the aromatic substance **122.**

Referring to the cross-sectional view of the aroma capsule **12** shown in FIG. 3, as stated above, the aromatic substance **122** is sealed in the aroma capsule **12** and the aroma capsule **12** is accommodated in the top accommodation open chamber **100** of the heating base **10** of the aroma-diffusing heating device **1.** When using the aroma-diffusing heating device **1,** the user simply needs to remove the sealing film **124** from the heat-transfer container **120** without contacting the aromatic substance **122.** Further, after the aromatic substance **122** is used up, the user can put a new aroma capsule in the top accommodation open chamber **100** of the heating base **10** of the aroma-diffusing heating device **1** as a substitute and needs not to wash the heat-transfer container **120** of the used aroma capsule **12.** Further, because the user does not need to clean the heat-transfer container **120,** the user needs not to worry about breaking down the heat-transfer container **120.** Further, the user needs not to prepare a container for holding essential oil. Further, different aroma capsules **12** designed to give off different good smells can be selectively used.

Further, the heat-transfer container **120** is configured to fit the shape of the top accommodation open chamber **100** of the heating base **10** of the aroma-diffusing heating device **1** so that the heat-transfer container **120** can be kept in close contact with the periphery of the top accommodation open chamber **100** of the heating base **10** of the aroma-diffusing heating device **1** to transfer heat energy from the heating base **10** to the aromatic substance **122** rapidly.

Further, the heating base **10** has a top flange **102** protruded from the top wall thereof around the top accommodation open chamber **100.** The top flange **102** has the inside wall thereof smoothly downwardly curved toward the top accommodation open chamber **100.** Thus, the user can insert the fingers along the smoothly downwardly curved inside wall of the top flange **102** to pick up the aroma capsule **12** from the top accommodation open chamber **100** conveniently. Further, the heat-transfer container **120** defines a rim **1202** around the top opening **1200.** The rim **1202** can be configured to extend in a horizontal or vertical manner relative to the elevation of the top opening **1200.** Thus, the user can grip the rim **1202** with the fingers to remove the aroma capsule **12** from the accommodation chamber **100** of the heating base **10** of the aroma-diffusing heating device **1** conveniently.

Further, the cap **14** defines a ventilation hole **140** for guiding fragrance out of the heat-transfer container **120** into the outside open air after removal of the sealing film **124** from the heat-transfer container **120.**

FIGS. 4-7 illustrate an alternate form of the aroma-diffusing heating device **1** in accordance with the present invention. According to this alternate form, the aroma capsule **12** can be used in a wall-mount night lamp, comprising a heat-transfer container **120** defining a top opening **1200** and a rim **1202** around the top opening **1200,** an aromatic substance (for example, aromatic wax) **122** held in the heat-transfer container **120,** a breathing film **126** having open spaces **1263** (for example, hole) therein selected from the material group of nonwoven fabric, fiber cloth, woven fabric, porous plastic membrane and porous silicon rubber and bonded with the border edge 1261 thereof to the heat-transfer container 120 above the aromatic substance 122(aromatic wax), and a sealing film 124 bonded to the rim 1202 of the heat-transfer container 120 to seal the aromatic substance 122(aromatic wax) and the breathing film 126 in the heat-transfer container 120, maintaining the quality of the aromatic substance 122(aromatic wax). Further, the border edge 1261 of the breathing film 126 can be bonded to the inside wall of the heat-transfer container 120 in the top opening 1200. Alternatively, the border edge 1261 of the breathing film 126 can be bonded to the rim 1202 of the heat-transfer container 120 above the top opening 1200.
When compared to conventional aromatic heating devices, the aroma-diffusing heating device 1 in accordance with the present invention uses a aroma capsule 12 that can be replaced by a new one when used up, and therefore, the user needs not to prepare an extra essential oil container or to worry about breaking down the heat-transfer container, and will not touch the essential oil during replacement of the essential oil.
Although particular embodiment of the invention have been described in detail for purposes of illustration, various modifications and enhancements may be made without departing from the scope of the invention. Accordingly, the invention is not to be limited except as by the appended claims.

## Claims

1. An aroma-diffusing heating device, comprising:
a heating base (10) defining a top accommodation open chamber (100), wherein the heating base (10) has a top flange (102) protruded from a top wall thereof around the top accommodation
open chamber (100), said top flange (102) having an inside wall thereof smoothly downwardly curved toward said top accommodation open chamber (100);
an aroma capsule (12) detachably accommodated in said top accommodation open chamber (100) and heatable by said heating base (10) to give off fragrance;
said aroma capsule (12) comprising a heat-transfer container (120) accommodated in said top accommodation open chamber (100) and configured to fit the shape of the top accommodation open chamber (100) of the heating base (10) so that the heat-transfer container (120) is kept in contact with the periphery of said top accommodation open chamber (100);
said heat-transfer container (120) defining a top opening (1200), and
an aromatic substance (122) held in said heat-transfer container (120);
said aroma capsule (12) comprising a breathing film (126) bonded to said heat-transfer container (120) to keep said aromatic substance (122) in said heat-transfer container (120),
said breathing film (126) having open spaces (1263) defined therein;
said aromatic substance (122) is an aromatic wax; and
a cap (14) capped on said heating base (10) over said aroma capsule (12).

2. The aroma-diffusing heating device as claimed in claim 1, wherein the aroma capsule (12) further comprises a sealing film (124) bonded to the heat-transfer container (120) to seal the aromatic substance (122) in the heat-transfer container (120), maintaining the quality of the aromatic substance (122).

3. The aroma-diffusing heating device as claimed in claim 1, wherein the aroma capsule (12) further comprises a sealing film (124); and
said heat-transfer container (120) comprise a rim (1202) around the top opening (1200);
the sealing film (124) bonded to the rim (1202) of the heat-transfer container (120) to seal the aromatic substance (122) in the heat-transfer container (120), maintaining the quality of the aromatic substance (122).

4. The aroma-diffusing heating device as claimed in claim 1, the breathing film (126) further comprises a border edge (1261), said breathing film (126) bonded with the border edge (1261) thereof to the heat-transfer container (120) above the aromatic substance (122).

5. The aroma-diffusing heating device as claimed in claim 1, the breathing film (126) further comprises a border edge 1261; the heat-transfer container (120) further comprise a rim (1202) around the top opening (1200);
said breathing film (126) bonded with the border edge (1261) thereof to the rim (1202) of the heat-transfer container (120) above the aromatic substance (122).

6. The aroma-diffusing heating device as claimed in claim 1, further comprising:
a sealing film (124) bonded to the heat-transfer container (120) to seal the aromatic substance (122) and the breathing film (126) in the heat-transfer container (120), maintaining the quality of the aromatic substance (122).

7. The aroma-diffusing heating device as claimed in claim 6, the heat-transfer container (120) further comprises a rim (1202) around the top opening (1200);
the sealing film (124) bonded to the rim (1202) of the heat-transfer container (120).

8. The aroma-diffusing heating device as claimed in claim 5, wherein the aroma capsule (12) further comprises a sealing film (124) bonded to the rim (1202) of the heat-transfer container (120) to seal the aromatic substance (122) and the breathing film (126) in the heat-transfer container (120), maintaining the quality of the aromatic substance (122).

9. The aroma-diffusing heating device as claimed in claim 1, wherein the heat-transfer container (120) is made of metal, ceramic, or plastic.

10. The aroma-diffusing heating device as claimed in claim 9, wherein the metal container is made of aluminum.

11. The aroma-diffusing heating device as claimed in claim 1 , wherein the breathing film (126) is selected from the material group consisting of nonwoven fabric, fiber cloth, woven fabric, porous plastic membrane or porous silicon rubber.

12. The aroma-diffusing heating device as claimed in one of the claims 1 - 11, comprising a ceramic bowl (16) supporting said heating base (10).

## Patentansprüche

1. Ein erhitzbarer Duftverdampfer, umfassend:
eine Heizbasis (10), die eine von oben aufnehmende offene Kammer (100) definiert, wobei die Heizbasis (10) einen oberen Flansch (102) aufweist, der von einer oberen Wandung um die oben aufnehmende offene Kammer (100) herum hervorsteht, wobei der besagte obere Flansch (102) eine Innenwand aufweist, die nach unten in Richtung der besagten von oben aufnehmenden offenen Kammer (100) sanft gekrümmt ist;
eine Duftkapsel (12), die in der besagten von oben aufnehmenden offenen Kammer (100) abnehmbar untergebracht und durch die besagte Heizbasis (10) beheizbar ist, um Duft abzugeben;
wobei die besagte Duftkapsel (12) einen Wärmeübertragungsbehälter (120) umfasst, der in der besagten von oben aufnehmenden offenen Kammer (100) untergebracht ist und so konfiguriert ist, dass er mit der Form der von oben aufnehmenden offenen Kammer (100) der Heizbasis (10) zusammenpasst, sodass der Wärmeübertragungsbehälter (120) mit dem Umfang der besagten von oben aufnehmenden offenen Kammer (100) in Kontakt gehalten wird;
wobei der besagte Wärmeübertragungsbehälter (120) eine obere Öffnung (1200) definiert,
wobei ein Duftstoff (122) im besagten Wärmeübertragungsbehälter (120) aufgenommen ist;
wobei die besagte Duftkapsel (12) eine atmungsaktive Folie (126) aufweist, die mit dem besagten Wärmeübertragungsbehälter (120) durch Kleben verbunden ist, um den besagten Duftstoff (122) im besagten Wärmeübertragungsbehälter (120) zu halten,
wobei die besagte atmungsaktive Folie (126) darin definierte offene Räume (1263) aufweist;
wobei der besagte Duftstoff (122) ein Duftwachs ist; und
einen Deckel (14), der auf die besagte Heizbasis (10) und über die besagte Duftkapsel (12) gedeckt ist.

2. Erhitzbarer Duftverdampfer nach Anspruch 1, worin die Duftkapsel (12) ferner eine Dichtungsfolie (124) umfasst, die mit dem Wärmeübertragungsbehälter (120) verbunden ist, um den Duftstoff (122) im Wärmeübertragungsbehälter (120) zu versiegeln, wodurch die Qualität des Duftstoffs (122) erhalten bleibt.

3. Erhitzbarer Duftverdampfer nach Anspruch 1, worin die Duftkapsel (12) ferner eine Dichtungsfolie (124) umfasst; wobei der besagte Wärmeübertragungsbehälter (120) einen Rand (1202) umfasst, der um die obere Öffnung (1200) herum angeordnet ist; wobei die Dichtungsfolie (124) mit dem Rand (1202) des Wärmeübertragungsbehälters (120) durch Kleben verbunden ist, um den Duftstoff (122) im Wärmeübertragungsbehälter (120) zu versiegeln, wodurch die Qualität des Duftstoffs (122) erhalten bleibt.

4. Erhitzbarer Duftverdampfer nach Anspruch 1, wobei die atmungsaktive Folie (126) ferner eine Randkante (1261) umfasst, wobei die besagte atmungsaktive Folie (126) mit der Randkante (1261) des Wärmeübertragungsbehälters (120) oberhalb des Duftstoffs (122) durch Kleben verbunden ist.

5. Erhitzbarer Duftverdampfer nach Anspruch 1, wobei die atmungsaktive Folie (126) ferner eine Randkante (1261) umfasst; wobei der Wärmeübertragungsbehälter (120) ferner einen Rand (1202) um die obere Öffnung (1200) herum aufweist; wobei die besagte atmungsaktive Folie (126) mit ihrer Randkante (1261) mit dem Rand (1202) des Wärmeübertragungsbehälters (120) oberhalb des Duftstoffs (122) durch Kleben verbunden ist.

6. Erhitzbarer Duftverdampfer nach Anspruch 1, ferner umfassend:
eine Dichtungsfolie (124), die mit dem Wärmeübertragungsbehälter (120) durch Kleben verbunden ist, um den Duftstoff (122) und die atmungsaktive Folie (126) im Wärmeübertragungsbehälter (120) zu versiegeln, wodurch die Qualität des Duftstoffs (122) erhalten bleibt.

7. Erhitzbarer Duftverdampfer nach Anspruch 6, wobei der Wärmeübertragungsbehälter (120) ferner einen Rand (1202) um die obere Öffnung (1200) herum aufweist; wobei die Dichtungsfolie (124) mit dem Rand (1202) des Wärmeübertragungsbehälters (120) durch Kleben verbunden ist.

8. Erhitzbarer Duftverdampfer nach Anspruch 5, worin die Duftkapsel (12) ferner eine mit dem Rand (1202) des Wärmeübertragungsbehälters (120) durch Kleben verbundene Dichtungsfolie (124) umfasst, um den Duftstoff (122) und die atmungsaktive Folie (126) im Wärmeübertragungsbehälter (120) zu versiegeln, wodurch die Qualität des Duftstoffs (122) erhalten bleibt.

9. Erhitzbarer Duftverdampfer nach Anspruch 1, worin der Wärmeübertragungsbehälter (120) aus Metall, Keramik oder Kunststoff hergestellt ist.

10. Erhitzbarer Duftverdampfer nach Anspruch 9, worin der Metallbehälter aus Aluminium hergestellt ist.

11. Erhitzbarer Duftverdampfer nach Anspruch 1, worin die atmungsaktive Folie (126) aus der aus Vliesstoff, Fasertuch, Gewebe, poröser Kunststoffmembran oder porösem Silikonkautschuk bestehenden Materialgruppe ausgewählt ist,.

12. Erhitzbarer Duftverdampfer nach eines der Ansprüche 1 bis 11, wobei dieser eine die besagte Heizbasis (10) stützende Keramikschale (16) umfasst.

## Revendications

1. Dispositif chauffant de diffusion d'arôme, **caractérisée par** le qu'elle comprend :
une base chauffante (10) définissant une chambre ouverte de logement supérieure (100), la base chauffante (10) présente une bride supérieure (102) en saillie à partir d'une paroi supérieure située autour de la chambre ouverte de logement supérieure (100), ladite bride supérieure (102) présentant une paroi interne doucement incurvée vers le bas vers ladite chambre ouverte de logement supérieure (100) ;
une capsule d'arôme (12) mobile logée dans ladite chambre ouverte de logement supérieure (100) et chauffable par ladite base chauffante (10) pour dégager le parfum ;
ladite capsule d'arôme (12) comprenant un récipient de transfert de chaleur (120) logé dans ladite chambre ouverte de logement supérieure(100) et configuré pour épouser la forme de la chambre ouverte de logement supérieure (100) de la base chauffante (10) de sorte que le récipient de transfert de chaleur (120) est maintenu en contact avec la périphérie de ladite chambre ouverte de logement supérieure(100) ;
ledit récipient de transfert de chaleur (120) définissant une ouverture supérieure (1200), et
une substance aromatique (122) contenue dans ledit récipient de transfert de chaleur (120) ;
ladite capsule d'arôme (12) comprenant un film respirant (126) collé au dit récipient de transfert de chaleur (120) pour conserver ladite substance aromatique (122) dans ledit récipient de transfert de chaleur (120),
ledit film respirant (126) présentant des espaces ouverts (1263) définis là ;
ladite substance aromatique (122) est une cire aromatique ; et
un capuchon (14) coiffant ladite base chauffante (10) au dessus de ladite capsule d'arôme (12).

2. Dispositif chauffant de diffusion d'arôme selon la revendication 1, **caractérisé par le fait que** la capsule d'arôme (12) comprend en outre un film d'étanchéité (124) collé au récipient de transfert de chaleur (120) pour enfermer la substance aromatique (122) dans le récipient de transfert de chaleur (120), maintenant la qualité de la substance aromatique (122).

3. Dispositif chauffant de diffusion d'arôme selon la revendication 1, **caractérisé par le fait que** la capsule d'arôme (12) comprend en outre un film d'étanchéité (124) ;
et
ledit récipient de transfert de chaleur (120) comprend un pourtour (1202) autour de l'ouverture supérieure (1200) ;
le film d'étanchéité (124) collé sur le pourtour (1202) du récipient de transfert de chaleur (120) pour enfermer la substance aromatique (122) dans le récipient de transfert de chaleur (120), maintenant la qualité de la substance aromatique (122).

4. Dispositif chauffant de diffusion d'arôme selon la revendication 1, **caractérisé par le fait que** le film respirant (126) comprend en outre une bordure (1261), ledit film respirant (126) avec sa bordure collée (1261) sur le récipient de transfert de chaleur (120) au dessus de la substance aromatique (122).

5. Dispositif chauffant de diffusion d'arôme selon la revendication 1, **caractérisé par le fait que** le film respirant (126) comprend en outre une bordure (1261) ;
le récipient de transfert de chaleur (120) comprend en outre un pourtour (1202) autour de l'ouverture supérieure (1200) ;
ledit film respirant (126) avec sa bordure collée (1261) sur le pourtour (1202) du récipient de transfert de chaleur (120) au dessus de la substance aromatique (122).

6. Dispositif chauffant de diffusion d'arôme selon la revendication 1, **caractérisée par** le qu'elle comprend :
un film d'étanchéité (124) collé au récipient de transfert de chaleur (120) pour enfermer la substance aromatique (122) et le film respirant (126) dans le récipient de transfert de chaleur (120), maintenant la qualité de la substance aromatique (122).

7. Dispositif chauffant de diffusion d'arôme selon la revendication 6, **caractérisé par le fait que** le récipient de transfert de chaleur (120) comprend en outre un pourtour (1202) autour de l'ouverture supérieure (1200) ;
le film d'étanchéité (124) collé sur le pourtour (1202) du récipient de transfert de chaleur (120).

8. Dispositif chauffant de diffusion d'arôme selon la revendication 5, **caractérisé par**
**le fait que** la capsule d'arôme (12) comprend en outre un film d'étanchéité (124) collé sur le pourtour (1202) du récipient de transfert de chaleur (120) pour enfermer la substance aromatique (122) et le film respirant (126) dans le récipient de transfert de chaleur (120), maintenant la qualité de la substance aromatique (122).

9. Dispositif chauffant de diffusion d'arôme selon la revendication 1, **caractérisé par**
**le fait que** le récipient de transfert de chaleur (120) est fabriqué en métal, céramique ou plastique.

10. Dispositif chauffant de diffusion d'arôme selon la revendication 9, **caractérisé par**
**le fait que** le récipient en métal est fabriqué en aluminium.

11. Dispositif chauffant de diffusion d'arôme selon la revendication 1, **caractérisé par**
**le fait que** le film respirant (126) est sélectionné à partir du groupe de matériaux consistant en tissu non tissé, tissu en fibres, tissu tissé, membrane en plastique poreux ou caoutchouc siliconé poreux.

12. Dispositif chauffant de diffusion d'arôme selon l'une des revendications 1-11,
**caractérisée par** le qu'elle comprend un bol en céramique (16) supportant ladite base chauffante (10).
